# EUROPEAN PATENT APPLICATION

(11) **EP 4 501 867 A1**
(43) Date of publication of application: **05.02.2025**
(21) Application number: 23774493.3
(22) Date of filing: 06.03.2023
(51) Int. Cl.: C02F 1/44, B01D 3/00, B01D 17/00, B01D 61/36, B01D 61/58, C12P 7/16

(54) **MEMBRANE SEPARATION SYSTEM AND OPERATION METHOD FOR MEMBRANE SEPARATION SYSTEM**

(30) Priority: 24.03.2022 JP 2022047921
(71) Applicant: NITTO DENKO CORPORATION, Ibaraki-shi Osaka 567-8680 (JP)
(72) Inventor: KIMURA, Naomichi, Ibaraki-shi, Osaka 567-8680 (JP); OGAWA, Tomoya, Ibaraki-shi, Osaka 567-8680 (JP); HIRAI, Tomoya, Ibaraki-shi, Osaka 567-8680 (JP)
(74) Representative: Hoffmann Eitle
(86) International application number: PCT/JP2023/008430
(87) International publication number: WO 2023/181894

(57) **Abstract**

The present invention provides a new membrane separation system suitable for efficiently separating a volatile organic compound from an aqueous solution containing the organic compound. A membrane separation system 100 of the present invention includes a first membrane separation device 10 having a first separation membrane 11, a separation vessel 25 for phase-separating an aqueous solution S2 into an aqueous phase and an organic phase, a second membrane separation device 30 having a second separation membrane 31, a first permeated fluid feed passage 51, and a second permeated fluid feed passage 55. The second permeated fluid feed passage 55 is connected to the second membrane separation device 30 and at least one selected from the group consisting of the first permeated fluid feed passage 51 and the separation vessel 25.

## Description

### TECHNICAL FIELD

The present invention relates to a membrane separation system and a method for operating a membrane separation system.

### BACKGROUND ART

There have been developed methods for producing a volatile organic compound (a fermented product), such as an alcohol, by fermenting a carbon source, such as glucose, by using a microorganism. The fermentation of a carbon source is carried out in an aqueous solution, for example. In this method, the fermentation by a microorganism stops in some cases when the content of the fermented product in the aqueous solution increases. In order to continue the production of the fermented product by a microorganism, it is necessary to separate the fermented product from the aqueous solution.

As an example of the method for separating a volatile organic compound from an aqueous solution containing the organic compound, a pervaporation method using a separation membrane can be mentioned. The pervaporation method is suitable for separating a volatile organic compound from an aqueous solution containing various substances. By combining a membrane separation device that performs the pervaporation method with a fermenter that produces a fermented product, it is possible to produce a fermented product continuously. For example, Patent Literature 1 discloses a membrane separation system obtained by combining a membrane separation device with a fermenter.

### CITATION LIST

### Patent Literature

Patent Literature 1: JP 2010-161987 A

### SUMMARY OF INVENTION

### Technical Problem

There is a need for a new membrane separation system suitable for efficiently separating a volatile organic compound from an aqueous solution containing the organic compound.

### Solution to Problem

The present invention provides a membrane separation system including:
a first membrane separation device having a first separation membrane that allows a first permeated fluid to be generated from an aqueous solution S1 containing a volatile organic compound, the first permeated fluid having a content of the organic compound higher than that in the aqueous solution S1;
a separation vessel for phase-separating an aqueous solution S2 containing the first permeated fluid into an aqueous phase having a content of water higher than that in the aqueous solution S2 and containing the organic compound and an organic phase having a content of the organic compound higher than that in the aqueous solution S2;
a second membrane separation device having a second separation membrane that allows a second permeated fluid to be generated from the aqueous phase, the second permeated fluid having a content of the organic compound higher than that in the aqueous phase;
a first permeated fluid feed passage connected to the first membrane separation device and the separation vessel and configured to supply the first permeated fluid from the first membrane separation device to the separation vessel; and
a second permeated fluid feed passage connected to the second membrane separation device and at least one selected from the group consisting of the first permeated fluid feed passage and the separation vessel and configured to supply the second permeated fluid from the second membrane separation device to the first permeated fluid feed passage and/or the separation vessel.

The present invention further provides a method for operating a membrane separation system including a first membrane separation device having a first separation membrane, a separation vessel, and a second membrane separation device having a second separation membrane, the method including:
generating, with the first separation membrane, a first permeated fluid from an aqueous solution S1 containing a volatile organic compound, the first permeated fluid having a content of the organic compound higher than that in the aqueous solution S1;
phase-separating, with the separation vessel, an aqueous solution S2 containing the first permeated fluid into an aqueous phase having a content of water higher than that in the aqueous solution S2 and containing the organic compound and an organic phase having a content of the organic compound higher than that in the aqueous solution S2;
generating, with the second separation membrane, a second permeated fluid from the aqueous phase, the second permeated fluid having a content of the organic compound higher than that in the aqueous phase; and
preparing the aqueous solution S2 by mixing the first permeated fluid with the second permeated fluid.

### Advantageous Effects of Invention

The present invention can provide a new membrane separation system suitable for efficiently separating a volatile organic compound from an aqueous solution containing the organic compound.

### BRIEF DESCRIPTION OF DRAWINGS

FIG. 1 is a schematic configuration diagram of a membrane separation system of Embodiment 1.
FIG. 2 is a schematic cross-sectional view showing an example of a first membrane separation device.
FIG. 3 is a schematic cross-sectional view of a separation membrane included in the first membrane separation device.
FIG. 4 is a schematic cross-sectional view showing an example of a second membrane separation device.
FIG. 5 is a perspective view schematically showing another example of the first membrane separation device.
FIG. 6 is a schematic configuration diagram of a membrane separation system of Embodiment 2.
FIG. 7 is a schematic configuration diagram of a membrane separation system of Calculation Example 1.
FIG. 8 is a schematic configuration diagram of a membrane separation system of Calculation Examples 13 and 14.

### DESCRIPTION OF EMBODIMENTS

A membrane separation system according to a first aspect of the present invention includes:
a first membrane separation device having a first separation membrane that allows a first permeated fluid to be generated from an aqueous solution S1 containing a volatile organic compound, the first permeated fluid having a content of the organic compound higher than that in the aqueous solution S1;
a separation vessel for phase-separating an aqueous solution S2 containing the first permeated fluid into an aqueous phase having a content of water higher than that in the aqueous solution S2 and containing the organic compound and an organic phase having a content of the organic compound higher than that in the aqueous solution S2;
a second membrane separation device having a second separation membrane that allows a second permeated fluid to be generated from the aqueous phase, the second permeated fluid having a content of the organic compound higher than that in the aqueous phase;
a first permeated fluid feed passage connected to the first membrane separation device and the separation vessel and configured to supply the first permeated fluid from the first membrane separation device to the separation vessel; and
a second permeated fluid feed passage connected to the second membrane separation device and at least one selected from the group consisting of the first permeated fluid feed passage and the separation vessel and configured to supply the second permeated fluid from the second membrane separation device to the first permeated fluid feed passage and/or the separation vessel.

According to a second aspect of the present invention, for example, in the membrane separation system according to the first aspect, the aqueous solution S2 is prepared by mixing the first permeated fluid with the second permeated fluid.

According to a third aspect of the present invention, for example, the membrane separation system according to the first or second aspect further includes a tank for storing the aqueous solution S1 to be supplied to the first membrane separation device.

According to a fourth aspect of the present invention, for example, in the membrane separation system according to the third aspect, the organic compound is a fermented product generated by a microorganism, and the tank is a fermenter for generating the organic compound.

According to a fifth aspect of the present invention, for example, in the membrane separation system according to the third or fourth aspect, the first separation membrane allows a first non-permeated fluid to be generated from the aqueous solution S1, the first non-permeated fluid having a content of the organic compound lower than that in the aqueous solution S1, the membrane separation system further includes a first non-permeated fluid discharge passage connected to the first membrane separation device and configured to discharge the first non-permeated fluid from the first membrane separation device, and the first non-permeated fluid discharge passage is connected to the tank.

According to a sixth aspect of the present invention, for example, in the membrane separation system according to any one of the first to fifth aspects, the second separation membrane allows a second non-permeated fluid to be generated from the aqueous phase, the second non-permeated fluid having a content of the organic compound lower than that in the aqueous phase, and the membrane separation system further includes a second non-permeated fluid discharge passage connected to the second membrane separation device and configured to discharge the second non-permeated fluid from the second membrane separation device.

According to a seventh aspect of the present invention, for example, the membrane separation system according to the sixth aspect further includes an aqueous solution feed passage connected to the first membrane separation device and configured to supply the aqueous solution S1 to the first membrane separation device, wherein the second non-permeated fluid discharge passage is connected to the aqueous solution feed passage.

According to an eighth aspect of the present invention, for example, in the membrane separation system according to the sixth aspect, the first membrane separation device has two membrane separation units and a connection passage connecting the two membrane separation units to each other, and the second non-permeated fluid discharge passage is connected to the connection passage.

According to a ninth aspect of the present invention, for example, in the membrane separation system according to any one of the sixth to eighth aspects, a content of the organic compound in the second non-permeated fluid is 2 wt% or less.

According to a tenth aspect of the present invention, for example, the membrane separation system according to any one of the first to ninth aspects further includes a distillation device that distills the organic phase and generates a distillate having a content of the organic compound lower than that in the organic phase.

According to an eleventh aspect of the present invention, for example, the membrane separation system according to the tenth aspect further includes a distillate discharge passage connected to the distillation device and configured to discharge the distillate from the distillation device, wherein the distillate discharge passage is connected to at least one selected from the group consisting of the first permeated fluid feed passage and the separation vessel.

According to a twelfth aspect of the present invention, for example, in the membrane separation system according to any one of the first to eleventh aspects, at least one selected from the group consisting of the first separation membrane and the second separation membrane is a pervaporation membrane.

According to a thirteenth aspect of the present invention, for example, in the membrane separation system according to any one of the first to twelfth aspects, the organic compound is at least one selected from the group consisting of an alcohol, ketone, and ester.

A method for operating a membrane separation system according to a fourteenth aspect of the present invention is a method for operating a membrane separation system including a first membrane separation device having a first separation membrane, a separation vessel, and a second membrane separation device having a second separation membrane, the method including:
generating, with the first separation membrane, a first permeated fluid from an aqueous solution S1 containing a volatile organic compound, the first permeated fluid having a content of the organic compound higher than that in the aqueous solution S1;
phase-separating, with the separation vessel, an aqueous solution S2 containing the first permeated fluid into an aqueous phase having a content of water higher than that in the aqueous solution S2 and containing the organic compound and an organic phase having a content of the organic compound higher than that in the aqueous solution S2;
generating, with the second separation membrane, a second permeated fluid from the aqueous phase, the second permeated fluid having a content of the organic compound higher than that in the aqueous phase; and
preparing the aqueous solution S2 by mixing the first permeated fluid with the second permeated fluid.

The present invention will be described in detail below. The following description is not intended to limit the present invention to a specific embodiment.

### <Embodiment 1>

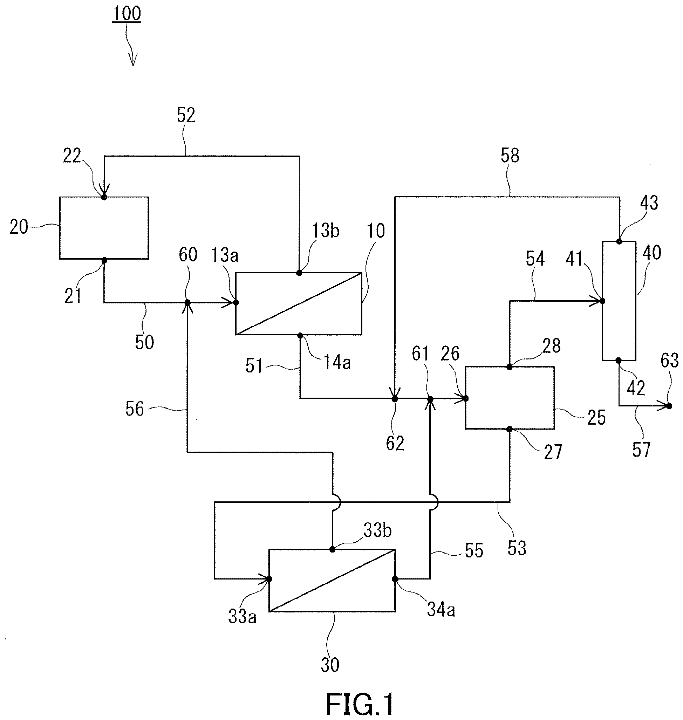

As shown in FIG. 1, a membrane separation system 100 of Embodiment 1 includes a first membrane separation device 10, a separation vessel 25, a second membrane separation device 30, a first permeated fluid feed passage 51, and a second permeated fluid feed passage 55.

The first membrane separation device 10 has a first separation membrane, and further has, for example, a feed space and a permeation space separated from each other by the first separation membrane. The first membrane separation device 10 is a device that performs membrane separation for an aqueous solution S1 containing a volatile organic compound C by using the first separation membrane. The first separation membrane that the first membrane separation device 10 has can separate the aqueous solution S1 into a first permeated fluid and a first non-permeated fluid. The first separation membrane is a membrane that allows the organic compound C contained in the aqueous solution S1 to preferentially permeate therethrough. Accordingly, the first permeated fluid separated by the first separation membrane has a content of the organic compound C higher than that in the aqueous solution S1. In contrast, the first non-permeated fluid has a content of the organic compound C lower than that in the aqueous solution S1.

The separation vessel 25 is a vessel for phase-separating an aqueous solution S2 containing the first permeated fluid. The aqueous solution S2 typically contains the later-described second permeated fluid in addition to the first permeated fluid. Specifically, the aqueous solution S2 is a mixed solution containing the first permeated fluid that is a liquid and the second permeated fluid that is a liquid. The aqueous solution S2 contains the organic compound C at a content higher than that in the aqueous solution S1. In one example, when the aqueous solution S2 is left at rest in the separation vessel 25, the aqueous solution S2 is phase-separated into an aqueous phase and an organic phase. The aqueous phase has a content of water higher than that in the aqueous solution S2 and contains the organic compound C. The organic compound C may be saturated in the aqueous phase. The organic phase has a content of the organic compound C higher than that in the aqueous solution S2. For example, the organic phase contains water in addition to the organic compound C and has a content of water lower than that in the aqueous solution S2.

The second membrane separation device 30 has a second separation membrane, and further has, for example, a feed space and a permeation space separated from each other by the second separation membrane. The second membrane separation device 30 is a device that performs membrane separation for the above-mentioned aqueous phase by using the second separation membrane. The second separation membrane that the second membrane separation device 30 has can separate the aqueous phase into a second permeated fluid and a second non-permeated fluid. The second separation membrane is a membrane that allows the organic compound C contained in the aqueous phase to preferentially permeate therethrough. Accordingly, the second permeated fluid separated by the second separation membrane has a content of the organic compound C higher than that in the aqueous phase. In contrast, the second non-permeated fluid has a content of the organic compound C lower than that in the aqueous phase.

The first permeated fluid feed passage 51 is a passage connected to a permeated fluid outlet (an outlet 14a) of the first membrane separation device 10 and a permeated fluid inlet (an inlet 26) of the separation vessel 25 and configured to deliver the first permeated fluid from the first membrane separation device 10 to the separation vessel 25. The first permeated fluid feed passage 51 may be provided with a decompression device and a heat exchanger (a chiller).

The decompression device provided to the first permeated fluid feed passage 51 can decompress an inside of the permeation space of the first membrane separation device 10, for example. Preferably, the decompression device is a vacuum device such as a vacuum pump. The vacuum pump is typically a gas transport vacuum pump, and a reciprocating vacuum pump and a rotary vacuum pump, etc. can be mentioned. Examples of the reciprocating vacuum pump include a diaphragm vacuum pump and a rocking piston vacuum pump. Examples of the rotary vacuum pump include: a liquid seal pump; an oil rotary pump (a rotary pump); a mechanical booster pump; and various kinds of dry pumps such as a roots dry pump, a claw dry pump, a screw dry pump, a turbo dry pump, and a scroll dry pump. The pump as the decompression device may include a variable speed mechanism for changing a rotational speed, etc. An example of the variable speed mechanism is an inverter that drives a motor of the pump. By controlling the rotational speed, etc. of the pump by the variable speed mechanism, it is possible to adjust properly a pressure in the permeation space of the first membrane separation device 10.

The heat exchanger provided to the first permeated fluid feed passage 51 can cool the first permeated fluid, for example. The heat exchanger is, for example, a gas-liquid heat exchanger that causes heat exchange between a cooling medium, such as an antifreeze, and the first permeated fluid that is a gas. The heat exchanger makes it possible to cool and condense the first permeated fluid even in the case where the first permeated fluid is a gas. That is, the heat exchanger can change the state of the first permeated fluid from a gaseous state to a liquid state. Note that the first permeated fluid feed passage 51 may be further provided with a heat exchanger for heating the condensed first permeated fluid that is a liquid.

The second permeated fluid feed passage 55 is connected to a permeated fluid outlet (an outlet 34a) of the second membrane separation device 30 and at least one selected from the group consisting of the first permeated fluid feed passage 51 and the separation vessel 25. In the example of FIG. 1, the second permeated fluid feed passage 55 is connected to the first permeated fluid feed passage 51 at a connecting point 61. The second permeated fluid feed passage 55 is a passage for supplying the second permeated fluid from the second membrane separation device 30 to the first permeated fluid feed passage 51 and/or the separation vessel 25.

The second permeated fluid is supplied to the first permeated fluid feed passage 51 or the separation vessel 25 via the second permeated fluid feed passage 55. Thereby, the first permeated fluid is mixed with the second permeated fluid to prepare the aqueous solution S2. The membrane separation system 100 of the present embodiment in which the aqueous solution S2 containing the first permeated fluid and the second permeated fluid is processed in the separation vessel 25 is suitable for efficiently separating the organic compound C from the aqueous solution S1.

In another aspect, the present invention provides a membrane separation system including:
the first membrane separation device 10 having the first separation membrane that allows the first permeated fluid to be generated from the aqueous solution S1 containing the volatile organic compound C, the first permeated fluid having a content of the organic compound C higher than that in the aqueous solution S1;
the separation vessel for phase-separating the aqueous solution S2 containing the first permeated fluid into the aqueous phase having a content of water higher than that in the aqueous solution S2 and containing the organic compound and the organic phase having a content of the organic compound C higher than that in the aqueous solution S2; and
the second membrane separation device 30 having the second separation membrane that allows the second permeated fluid to be generated from the aqueous phase, the second permeated fluid having a content of the organic compound C higher than that in the aqueous phase, wherein the aqueous solution S2 is prepared by mixing the first permeated fluid with the second permeated fluid.

The second permeated fluid feed passage 55 may be provided with a decompression device and a heat exchanger (a chiller). The decompression device provided to the second permeated fluid feed passage 55 can decompress an inside of the permeation space of the second membrane separation device 30, for example. As the decompression device, those described above can be used. The heat exchanger provided to the second permeated fluid feed passage 55 can cool the second permeated fluid, for example. The heat exchanger is, for example, a gas-liquid heat exchanger that causes heat exchange between a cooling medium, such as an antifreeze, and the second permeated fluid that is a gas. The heat exchanger makes it possible to cool and condense the second permeated fluid even in the case where the second permeated fluid is a gas. That is, the heat exchanger can change the state of the second permeated fluid from a gaseous state to a liquid state.

Note that the second permeated fluid feed passage 55 may not be provided with the decompression device or the heat exchanger. In one example, the inside of the permeation space of the second membrane separation device 30 may be decompressed using the decompression device provided to the first permeated fluid feed passage 51. That is, one decompression device may be shared by the first membrane separation device 10 and the second membrane separation device 30. In this case, the decompression device is preferably located, on the first permeated fluid feed passage 51, between the connecting point 61 and the separation vessel 25.

Similarly, the second permeated fluid may be cooled by the heat exchanger provided to the first permeated fluid feed passage 51. That is, one heat exchanger may be shared for the first permeated fluid and the second permeated fluid. Specifically, the aqueous solution S2 may be prepared by cooling a gas mixture containing the first permeated fluid that is a gas and the second permeated fluid that is a gas with the heat exchanger provided to the first permeated fluid feed passage 51. In this case, the heat exchanger is preferably located, on the first permeated fluid feed passage 51, between the connecting point 61 and the separation vessel 25.

Note that, as described later, the organic compound C may be a fermented product generated by fermentation of a carbon source by a microorganism, or may be an alcohol (a bioalcohol, especially biobutanol), such as n-butanol, generated by a microorganism. That is, the aqueous solution S1 may be a fermented liquid containing the organic compound C as the fermented product.

In another aspect, the present invention provides a membrane separation system including:
the first membrane separation device 10 having the first separation membrane that allows the first permeated fluid to be generated from the aqueous solution S1 containing the volatile organic compound C, the first permeated fluid having a content of the organic compound C higher than that in the aqueous solution S1;
the separation vessel for phase-separating the aqueous solution S2 containing the first permeated fluid into the aqueous phase having a content of water higher than that in the aqueous solution S2 and containing the organic compound and the organic phase having a content of the organic compound C higher than that in the aqueous solution S2; and
the second membrane separation device 30 having the second separation membrane that allows the second permeated fluid to be generated from the aqueous phase, the second permeated fluid having a content of the organic compound C higher than that in the aqueous phase, wherein
the organic compound C is a fermented product generated by a microorganism.

The membrane separation system 100 further includes a tank 20. The tank 20 stores the aqueous solution S1 to be supplied to the first membrane separation device 10. The tank 20 is typically a fermenter for generating the organic compound C by fermentation of a carbon source by a microorganism.

The membrane separation system 100 further includes an aqueous solution feed passage 50 and a first non-permeated fluid discharge passage 52. The aqueous solution feed passage 50 is a passage connected to an aqueous solution outlet (an outlet 21) of the tank 20 and an aqueous solution inlet (an inlet 13a) of the first membrane separation device 10 and configured to supply the aqueous solution S1 from the tank 20 to the first membrane separation device 10. The aqueous solution feed passage 50 may be provided with a pump that controls a flow rate of the aqueous solution S1.

The first non-permeated fluid discharge passage 52 is a passage connected to a non-permeated fluid outlet (an outlet 13b) of the first membrane separation device 10 and configured to discharge the first non-permeated fluid from the first membrane separation device 10. The first non-permeated fluid discharge passage 52 may be connected to a non-permeated fluid inlet (an inlet 22) of the tank 20 and configured to deliver the first non-permeated fluid to the tank 20. That is, the membrane separation system 100 may be configured to allow the first non-permeated fluid to be mixed with the aqueous solution S1 in the tank 20 and circulate through the aqueous solution feed passage 50 and the first non-permeated fluid discharge passage 52. In the case where the first non-permeated fluid is delivered to the tank 20, the aqueous solution S1 is mixed with the first non-permeated fluid and the content of the organic compound C in the aqueous solution S1 decreases in the tank 20. In the case where the tank 20 is a fermenter, a decrease in the content of the organic compound C in the aqueous solution S1 can inhibit fermentation by a microorganism from stopping, thereby making it possible to produce the fermented product continuously.

The membrane separation system 100 further includes an aqueous phase feed passage 53. The aqueous phase feed passage 53 is a passage connected to an aqueous phase outlet (an outlet 27) of the separation vessel 25 and an aqueous phase inlet (an inlet 33a) of the second membrane separation device 30 and configured to supply the aqueous phase from the separation vessel 25 to the second membrane separation device 30. The aqueous phase feed passage 53 may be provided with a pump that controls a flow rate of the aqueous phase.

The membrane separation system 100 further includes a second non-permeated fluid discharge passage 56. The second non-permeated fluid discharge passage 56 is a passage connected to a non-permeated fluid outlet (an outlet 33b) of the second membrane separation device 30 and configured to discharge the second non-permeated fluid from the second membrane separation device 30. The second non-permeated fluid discharge passage 56 may be connected to the aqueous solution feed passage 50 at a connecting point 60 and configured to deliver the second non-permeated fluid to the aqueous solution feed passage 50. That is, the membrane separation system 100 may be configured to allow the second non-permeated fluid to be mixed with the aqueous solution S1 in the aqueous solution feed passage 50. Such a configuration makes it possible to recycle the second non-permeated fluid.

The membrane separation system 100 may further include a distillation device 40 that distills the organic phase generated in the separation vessel 25. The distillation device 40 makes it possible to further increase the content of the organic compound C in the organic phase (a residue) by heating the water together with the organic compound C. A distillate obtained by distilling the organic phase has a content of the organic compound C lower than that in the organic phase supplied to the distillation device 40 and a content of water higher than that in the organic phase. The distillation device 40 has a configuration for heating the organic phase, for example. The distillation device 40 is typically a distillation column.

The membrane separation system 100 further includes an organic phase feed passage 54, for example. The organic phase feed passage 54 is a passage connected to an organic phase outlet (an outlet 28) of the separation vessel 25 and an organic phase inlet (an inlet 41) of the distillation device 40 and configured to supply the organic phase from the separation vessel 25 to the distillation device 40. The organic phase feed passage 54 may be provided with a pump that controls a flow rate of the organic phase.

The membrane separation system 100 further includes, for example, a residue discharge passage 57 and a distillate discharge passage 58. The residue discharge passage 57 is a passage connected to a residue outlet (an outlet 42) of the distillation device 40 and configured to discharge the residue (the organic phase after being distilled) from the distillation device 40. In the residue discharge passage 57, an opening (a discharge outlet 63) for discharging the residue from the residue discharge passage 57 is formed. The residue discharge passage 57 may be connected to a tank to store the residue.

The distillate discharge passage 58 is a passage connected to a distillate outlet (an outlet 43) of the distillation device 40 and configured to discharge the distillate from the distillation device 40. The distillate discharge passage 58 may be connected to at least one selected from the group consisting of the first permeated fluid feed passage 51 and the separation vessel 25. In the example of FIG. 1, the distillate discharge passage 58 is connected to the first permeated fluid feed passage 51 at a connecting point 62. In such a manner, the membrane separation system 100 may be configured to allow the distillate to be mixed with the first permeated fluid and the second permeated fluid in the first permeated fluid feed passage 51 and the separation vessel 25. Such a configuration makes it possible to recycle the distillate. In the case where the distillate discharge passage 58 is connected to the first permeated fluid feed passage 51 and the separation vessel 25, the aqueous solution S2 contains the first permeated fluid, the second permeated fluid, and the distillate. Note that the connecting point 62 is located, on the first permeated fluid feed passage 51, between the first membrane separation device 10 and the connecting point 61, for example.

The membrane separation system 100 may further include a controller (not shown) that controls each member of the membrane separation system 100. The controller is, for example, a DSP (Digital Signal Processor) including an A/D conversion circuit, an input/output circuit, an arithmetic circuit, a storage device, etc. A program for operating properly the membrane separation system 100 is stored in the controller.

Each passage of the membrane separation system 100 is composed of, for example, a metal or resin pipe unless otherwise noted. Each passage may be provided with a sensor, etc. for measuring the content of the organic compound C as necessary.

### [First membrane separation device]

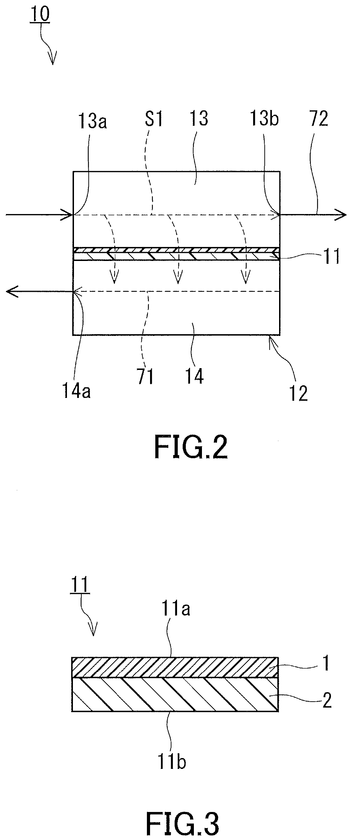

As shown in FIG. 2, the first membrane separation device 10 includes a first separation membrane 11 and a tank 12. The tank 12 has a first chamber 13 and a second chamber 14. The first chamber 13 functions as the feed space to which the aqueous solution S1 is supplied. The second chamber 14 functions as the permeation space to which a first permeated fluid 71 is supplied. The first permeated fluid 71 is obtained by allowing the aqueous solution S1 to permeate through the first separation membrane 11. The first separation membrane 11 is disposed in the tank 12. In the tank 12, the first separation membrane 11 separates the first chamber 13 and the second chamber 14 from each other. The first separation membrane 11 extends from one of a pair of wall surfaces of the tank 12 to the other.

The first chamber 13 has the inlet 13a and the outlet 13b. The second chamber 14 has the outlet 14a. The inlet 13a of the first chamber 13 is an opening for supplying the aqueous solution S1 to the first membrane separation device 10. The outlet 14a of the second chamber 14 is an opening for discharging the first permeated fluid 71 from the first membrane separation device 10. The outlet 13b of the first chamber 13 is an opening for discharging, from the first membrane separation device 10, the aqueous solution S1 (a first non-permeated fluid 72) not having permeated through the first separation membrane 11. The inlet 13a, the outlet 13b, and the outlet 14a are formed, for example, in wall surfaces of the tank 12.

The first membrane separation device 10 is suitable for a flow-type (continuous-type) membrane separation method. However, the first membrane separation device 10 may be used for a batch-type membrane separation method.

### (First separation membrane)

The first separation membrane 11 is not particularly limited as long as the first separation membrane 11 allows the organic compound C contained in the aqueous solution S1 to preferentially permeate therethrough. The first separation membrane 11 is, for example, a pervaporation membrane that allows the first permeated fluid 71 that is a gas to be generated by a pervaporation method.

As shown in FIG. 3, the first separation membrane 11 includes, for example, a separation functional layer 1 and a porous support member 2 supporting the separation functional layer 1. The first separation membrane 11 may further include a protective layer (not shown) that protects the separation functional layer 1. The separation functional layer 1 is in direct contact with the porous support member 2, for example. For example, the first separation membrane 11 has a principal surface 11a, on the separation functional layer side, that is exposed to the first chamber 13 and a principal surface 11b, on the porous support member side, that is exposed to the second chamber 14.

### (Separation functional layer)

The separation functional layer 1 is a layer that allows the organic compound C contained in the aqueous solution S1 to preferentially permeate therethrough. The separation functional layer 1 includes a hydrophobic material, for example. In the present description, the term "hydrophobic material" refers to, for example, a material that has a static contact angle exceeding 90° with respect to water when a 10 µL drop of the water (temperature 25°C) is dropped on a surface of a specimen composed of the material. Note that the static contact angle with respect to water can be measured using a commercially available contact angle meter.

Examples of the hydrophobic material include a compound having a siloxane bond (a Si-O-Si bond), an olefin-based polymer, an oil, and a fluorine-based compound. The separation functional layer 1 preferably includes a compound having a siloxane bond as the hydrophobic material. The compound having a siloxane bond is typically a silicone-based polymer. The silicone-based polymer may be a solid or a liquid at 25°C. Specific examples of the silicone-based polymer include polydimethylsiloxane (PDMS). Specific examples of the olefin-based polymer include polypropylene. Examples of the oil include a hydrocarbon-based oil such as liquid paraffin. Examples of the fluorine-based compound include polytetrafluoroethylene (PTFE), polyvinylidene fluoride (PVDF), and tetrafluoroethylene-perfluoroalkyl vinyl ether copolymer (PFA). These hydrophobic materials can be used alone or two or more of them can be used in combination.

The separation functional layer 1 may include the hydrophobic material as a main component, or may be composed substantially of the hydrophobic material alone. The term "main component" means a component having a largest content in the separation functional layer 1 in terms of weight ratio.

The separation functional layer 1 may include a matrix including the hydrophobic material and a filler dispersed in the matrix. The filler is buried in the matrix. In the matrix, all particles of the filler may be spaced from each other or may aggregate partially.

The filler includes, for example, an inorganic material such as zeolite, silica, or bentonite. The zeolite included in the filler is preferably a high-silica zeolite having a high ratio of silica with respect to alumina. Having high resistance to hydrolysis, the high-silica zeolite is suitably used for separating the aqueous solution S1. As the high-silica zeolite, HSZ (registered trademark) available from Tosoh Corporation, HiSiv (registered trademark) available from UNION SHOWA K.K., USKY available from UNION SHOWA K.K., and Zeoal (registered trademark) available from Nakamura Choukou Co., Ltd. can be used.

The filler may include a metal organic framework (MOF). The metal organic framework is also referred to as a porous coordination polymer (PCP). The metal organic framework is preferably hydrophobic. The metal organic framework includes a metal ion and an organic ligand, for example. Examples of the metal ion include a Zn ion. The organic ligand includes an aromatic ring, for example. Examples of the aromatic ring included in the organic ligand include an imidazole ring. Examples of the organic ligand include 2-methylimidazole. Specific examples of the metal organic framework include ZIF-8.

The filler is in the form of particles, for example. The term "form of particles" herein includes spherical, elliptical, flaky, and fibrous forms. An average particle diameter of the filler is, for example, but not particularly limited to, 50 µm or less, preferably 20 µm or less, and more preferably 10 µm or less. The lower limit of the average particle diameter of the filler is 0.01 µm, for example. The average particle diameter of the filler can be determined by the following method, for example. First, a cross-section of the separation functional layer 1 is observed using a transmission electron microscope. The area of a specific particle of the filler on the resulting electron microscope image is calculated by image processing. The diameter of a circle having the same area as the calculated area is regarded as the particle diameter (the diameter of the particle) of the specific filler particle. The particle diameter was calculated for any number (at least 50) of the filler particles, and the average of the calculated values was regarded as the average particle diameter of the filler.

The content of the filler in the separation functional layer 1 is, for example, 10 wt% or more, preferably 30 wt% or more, and more preferably 40 wt% or more. The upper limit of the content of the filler in the separation functional layer 1 is, for example, but not particularly limited to, 70 wt%. The content of the matrix in the separation functional layer 1 is, for example, but not particularly limited to, 30 wt% to 90 wt%.

The separation functional layer 1 has a thickness of, for example, 200 µm or less, preferably 100 µm or less, and more preferably 80 µm or less. The separation functional layer 1 may have a thickness of 1.0 µm or more, 10 µm or more, and 30 µm or more.

The separation functional layer 1 may have a microporous structure with an average pore diameter of less than 0.01 µm, but may be a dense layer having no pore on its surface.

### (Porous support member)

Examples of the porous support member 2 include: a nonwoven fabric; porous polytetrafluoroethylene; aromatic polyamide fiber; a porous metal; a sintered metal; porous ceramic; porous polyester; porous nylon; activated carbon fiber; latex; silicone; silicone rubber; a permeable (porous) polymer including at least one selected from the group consisting of polyvinyl fluoride, polyvinylidene fluoride, polyurethane, polypropylene, polyethylene, polystyrene, polycarbonate, polysulfone, polyether ether ketone, polyacrylonitrile, polyimide, and polyphenylene oxide; a metallic foam having an open cell or a closed cell; a polymer foam having an open cell or a closed cell; silica; a porous glass; and a mesh screen. The porous support member 2 may be a combination of two or more of these materials.

The porous support member 2 has an average pore diameter of 0.01 to 0.4 µm, for example. A thickness of the porous support 2 is, for example, but not particularly limited to, 10 µm or more, preferably 50 µm or more, and more preferably 100 µm or more. The thickness of the porous support 2 is, for example, 300 µm or less, and preferably 200 µm or less.

### (Protective layer)

The protective layer covers a surface of the separation functional layer 1, for example. A material of the protective layer is, for example, but not particularly limited to, a silicone resin. The material of the protective layer may be the same as that of the matrix of the separation functional layer 1. A thickness of the protective layer is, for example, but not particularly limited to, 5 µm or more, preferably 10 µm or more, and more preferably 20 µm or more. The thickness of the protective layer is, for example, 100 µm or less, and preferably 50 µm or less.

### (Method for producing first separation membrane)

The first separation membrane 11 can be produced by, for example, forming the separation functional layer 1 on the porous support member 2. Specifically, a coating liquid containing the materials of the separation functional layer 1 is prepared first. The coating liquid may contain, in addition to the filler, a dispersant for dispersing the filler in the coating liquid. In the case where the coating liquid contains the compound having a siloxane bond, the coating liquid may further contain a catalyst for curing the compound. Next, the coating liquid is applied onto the porous support 2 to obtain a coating. The coating is dried to obtain the separation functional layer 1.

### (Characteristics of first separation membrane)

A separation factor of the first separation membrane 11 for the organic compound C with respect to water is not particularly limited. In one example, a separation factor α of the first separation membrane 11 for n-butanol (BuOH) with respect to water may be 10 to 100. The separation factor α can be measured by the following method. First, in a state in which a liquid mixture composed of BuOH and water is in contact with one surface (the principal surface 11a, on the separation functional layer side, of the first separation membrane 11, for example) of the first separation membrane11, a space adjacent to another surface (the principal surface 11b, on the porous support member side, of the first separation membrane 11, for example) of the first separation membrane 11 is decompressed. Thereby, a permeated fluid having permeated through the first separation membrane 11 can be obtained. A weight ratio of the water and a weight ratio of the BuOH in the permeated fluid are measured. In the above procedure, the content of the BuOH in the liquid mixture is 0.5 wt%. The liquid mixture in contact with the first separation membrane 11 has a temperature of 30°C. The space adjacent to the other surface of the first separation membrane 11 is decompressed to 1.5 kPa. The separation factor α can be calculated by the following formula. Note that in the following formula, X_{A} and X_{B} are respectively a weight ratio of the BuOH and a weight ratio of the water in the liquid mixture. Y_{A} and Y_{B} are respectively the weight ratio of the BuOH and the weight ratio of the water in the permeated fluid having permeated through the first separation membrane 11. Separation factor α = (Y_{A}/Y_{B})/(X_{A}/X_{B})

Under the above measurement conditions for the separation factor α, a flux of the BuOH permeating through the first separation membrane 11 is, for example, but not particularly limited to, 0.01 (g/min/m²) to 1.0 (g/min/m²).

### [Second membrane separation device]

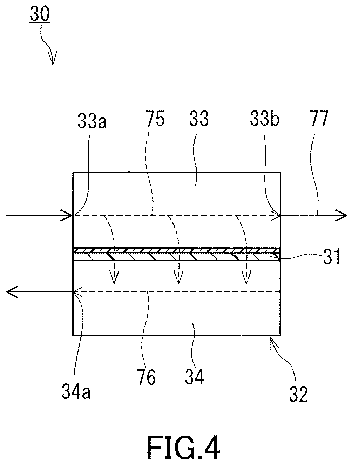

As shown in FIG. 4, the second membrane separation device 30 includes a second separation membrane 31 and a tank 32. The tank 32 has a third chamber 33 and a fourth chamber 34. The third chamber 33 functions as the feed space to which an aqueous phase 75 obtained in the separation vessel 25 is supplied. The fourth chamber 34 functions as the permeation space to which a second permeated fluid 76 is supplied. The second permeated fluid 76 is obtained by allowing the aqueous phase 75 to permeate through the second separation membrane 31. The second separation membrane 31 is disposed in the tank 32. In the tank 32, the second separation membrane 31 separates the third chamber 33 and the fourth chamber 34 from each other. The second separation membrane 31 extends from one of a pair of wall surfaces of the tank 32 to the other.

The third chamber 33 has the inlet 33a and the outlet 33b. The fourth chamber 34 has the outlet 34a. The inlet 33a of the third chamber 33 is an opening for supplying the aqueous phase 75 to the second membrane separation device 30. The outlet 34a of the fourth chamber 34 is an opening for discharging the second permeated fluid 76 from the second membrane separation device 30. The outlet 33b of the third chamber 33 is an opening for discharging, from the second membrane separation device 30, the aqueous phase 75 (a second non-permeated fluid 77) not having permeated through the second separation membrane 31. The inlet 33a, the outlet 33b, and the outlet 34a are formed, for example, in wall surfaces of the tank 32.

The second membrane separation device 30 is suitable for a flow-type (continuous-type) membrane separation method. However, the second membrane separation device 30 may be used for a batch-type membrane separation method.

The second separation membrane 31 may or may not be the same as the first separation membrane 11 except for its membrane area. The second separation membrane 31 is, for example, a pervaporation membrane that allows the second permeated fluid 76 that is a gas to be generated by a pervaporation method. In one example, at least one selected from the group consisting of the first separation membrane 11 and the second separation membrane 31 is a pervaporation membrane, and it is preferable that the first separation membrane 11 and the second separation membrane 31 be each a pervaporation membrane.

### [Method for operating membrane separation system]

A method for operating the membrane separation system 100 of the present embodiment includes: generating, with the first separation membrane 11, the first permeated fluid 71 from the aqueous solution S1 (a first separation step); phase-separating, with the separation vessel 25, the aqueous solution S2 into an aqueous phase and an organic phase (a second separation step); generating, with the second separation membrane 31, the second permeated fluid 76 from the aqueous phase (a third separation step); and preparing the aqueous solution S2 by mixing the first permeated fluid 71 with the second permeated fluid 76 (a preparation step).

### (First separation step)

The first separation step is performed as follows, for example. First, the aqueous solution S1 is supplied to the first chamber 13 of the first membrane separation device 10 via the aqueous solution feed passage 50. This makes it possible to bring the aqueous solution S1 into contact with one surface (the principal surface 11a, for example) of the first separation membrane 11.

The organic compound C contained in the aqueous solution S1 is not particularly limited as long as it has volatility. In the present description, the term "organic compound having volatility" refers to an organic compound that has a boiling point of 20°C to 260°C, preferably 50 to 260°C, under an atmospheric pressure (101.325 kPa). Note that the organic compound C allows, for example, an aqueous phase and an organic phase to be generated when a concentration of the organic compound C is high in the aqueous solution.

The number of carbon atoms in the organic compound C is, for example, but not particularly limited to, 10 or less, and may be 8 or less, 6 or less, or even 4 or less. The lower limit of the number of carbon atoms in the organic compound C may be 1 or 2. The organic compound C has, for example, a functional group including an oxygen atom, such as a hydroxyl group, a carbonyl group, an ether group, and an ester group. In the organic compound C, the number of the functional groups including an oxygen atom is typically one.

The organic compound C is, for example, at least one selected from the group consisting of an alcohol, ketone, and ester, and preferred is an alcohol. The alcohol may be an alkyl alcohol composed only of an alkyl group and a hydroxyl group, or may be an aryl alcohol including an aryl group and a hydroxyl group. The alkyl alcohol may be linear, branched, or cyclic. Examples of the alkyl alcohol include n-propanol, n-butanol (BuOH), 2-butanol, isobutanol, t-butanol, and n-pentanol, and preferred is n-butanol. Examples of the aryl alcohol include phenol.

The ketone may be dialkylketone composed only of an alkyl group and a carbonyl group. Examples of the dialkylketone include methyl ethyl ketone (MEK) and acetone.

The ester may be aliphatic alkylester composed only of an alkyl group and an ester group. Examples of the aliphatic alkylester include ethyl acetate.

Note that the organic compound C is not limited to those mentioned above. The organic compound C may be aromatic hydrocarbon such as benzene, toluene, or xylene.

The aqueous solution S1 may contain one kind of the organic compound C, or two or more kinds of the organic compounds C. The content of the organic compound C in the aqueous solution S1 is, for example, 50 wt% or less, and may be 30 wt% or less, 10 wt% or less, 5 wt% or less, 2 wt% or less, or even 1 wt% or less. The lower limit of the content of the organic compound C is, for example, but not particularly limited to, 0.01 wt%.

The organic compound C may be a fermented product generated by fermentation of a carbon source by a microorganism, or may be an alcohol (a bioalcohol, especially biobutanol), such as n-butanol, generated by a microorganism. That is, the aqueous solution S1 may be a fermented liquid containing the organic compound C as the fermented product. However, the aqueous solution S1 is not limited to the fermented liquid and may be waste water discharged from a chemical plant, etc.

The aqueous solution S1 may further contain an additional component, such as a microorganism, a carbon source, a nitrogen source, and an inorganic ion, other than water and the organic compound C. Examples of the microorganism include a bacteria such as Clostridium. Examples of the carbon source include polysaccharides such as starch, and monosaccharides such as glucose. The content of the additional component in the aqueous solution S1 is, for example, but not particularly limited to, 1 wt% to 10 wt%.

The amount of the aqueous solution S1 to be supplied to the first membrane separation device 10 is not particularly limited and is determined according to the processing capacity of the first membrane separation device 10. In one example, the amount of the aqueous solution S1 to be supplied is 0.5 kg/hr to 500 kg/hr. The aqueous solution S1 to be supplied to the first chamber 13 may be heated in advance. In one example, the aqueous solution S1 to be supplied to the first chamber 13 has a temperature of 30°C to 75°C.

Next, in a state in which the aqueous solution S1 is in contact with the one surface of the first separation membrane 11, a space adjacent to another surface (the principal surface 11b, for example) of the first separation membrane 11 is decompressed. Specifically, an inside of the second chamber 14 is decompressed via the outlet 14a. The inside of the second chamber 14 can be decompressed by, for example, the decompression device provided to the first permeated fluid feed passage 51. A pressure in the second chamber 14 is, for example, 50 kPa or less, and may be 20 kPa or less, 10 kPa or less, 5 kPa or less, 3 kPa or less, or even 2 kPa or less. In the present description, the term "pressure" means an absolute pressure unless otherwise noted.

Decompressing the inside of the second chamber 14 makes it possible to obtain, on the side of the other surface of the first separation membrane 11, the first permeated fluid 71 having a high content of the organic compound C. In other words, the first permeated fluid 71 is supplied to the second chamber 14. The first permeated fluid 71 may be a gas or a liquid in the second chamber 14. The first permeated fluid 71 is discharged outside the first membrane separation device 10 via the outlet 14a. The first permeated fluid 71 that is a gas is cooled by, for example, the heat exchanger provided to the first permeated fluid feed passage 51, or the like. Thereby, the first permeated fluid 71 is liquefied to obtain the first permeated fluid 71 that is a liquid.

On the other hand, the content of the organic compound C in the aqueous solution S1 gradually decreases from the inlet 13a toward the outlet 13b in the first chamber 13. The aqueous solution S1 processed in the first chamber 13 (the first non-permeated fluid 72) is discharged outside the first membrane separation device 10 via the outlet 13b. The first non-permeated fluid 72 is typically a liquid.

As described above, the first separation membrane 11 of the first membrane separation device 10 allows the organic compound C contained in the aqueous solution S1 to preferentially permeate therethrough. Accordingly, the first permeated fluid 71 obtained by the operation of the first membrane separation device 10 has a content of the organic compound C higher than that in the aqueous solution S1 to be supplied to the first membrane separation device 10. A ratio of the content (wt%) of the organic compound C in the first permeated fluid 71 with respect to the content (wt%) of the organic compound C in the aqueous solution S1 is, for example, but not particularly limited to, 5 to 50.

### (Second separation step)

The second separation step is performed as follows, for example. First, the aqueous solution S2 prepared by the later-described preparation step is supplied to the separation vessel 25, or the aqueous solution S2 is prepared in the separation vessel 25 in the preparation step. The aqueous solution S2 is an aqueous solution containing the organic compound C. The content of the organic compound C in the aqueous solution S2 is not particularly limited as long as the aqueous solution S2 can be phase-separated into an aqueous phase and an organic phase, and it is, for example, 7 wt% or more, and may be 8 wt% or more, 10 wt% or more, 20 wt% or more, 30 wt% or more, or even 50 wt% or more. The upper limit of the content of the organic compound C in the aqueous solution S2 is, for example, but not particularly limited to, 80 wt%. For example, in the case where the organic compound C is n-butanol, the aqueous solution S2 can be phase-separated into an aqueous phase and an organic phase when the content of the n-butanol in the aqueous solution S2 is 7 wt% or more.

Next, the aqueous solution S2 is left at rest in the separation vessel 25. Thereby, the aqueous solution S2 is phase-separated into an aqueous phase and an organic phase. As described above, the aqueous phase has a content of water higher than that in the aqueous solution S2 and contains the organic compound C. The aqueous phase contains, for example, water as a main component, and the content of the water is 50 wt% to 95wt%, for example. The content of the organic compound C in the aqueous phase is lower than the content of the organic compound C in the aqueous solution S2 and is, for example, less than 50 wt%, and may be 30 wt% or less, 10 wt% or less, or even 7 wt% or less. The organic compound C may be saturated in the aqueous phase. In the case where the organic compound C is n-butanol, the content of the n-butanol in the aqueous phase may be approximately 7 wt%.

The content of the organic compound C in the organic phase is higher than the content of the organic compound C in the aqueous solution S2 and is, for example, 50 wt% or more, and may be 60 wt% or more, 70 wt% or more, or even80 wt% or more. The organic phase contains water in addition to the organic compound C, for example. The content of the water in the organic phase is lower than the content of water in the aqueous solution S2 and is, for example, less than 50 wt%, and may be 40 wt% or less, 30 wt% or less, or even 20 wt% or less.

Next, the aqueous phase is discharged outside the separation vessel 25 via the outlet 27. The organic phase is discharged outside the separation vessel 25 via the outlet 28. The operating method of the present embodiment makes it possible to obtain the organic compound C at a relatively high purity by recovering the organic phase. However, as described later, the organic phase may also be distilled in the operating method of the present embodiment in order to increase the content of the organic compound C in the organic phase.

### (Third separation step)

The third separation step is performed as follows, for example. First, the aqueous phase 75 obtained in the second separation step is supplied to the third chamber 33 of the second membrane separation device 30 via the aqueous phase feed passage 53. This makes it possible to bring the aqueous phase 75 into contact with one surface of the second separation membrane 31. The amount of the aqueous phase 75 to be supplied to the second membrane separation device 30 is not particularly limited and is determined according to the processing capacity of the second membrane separation device 30. In one example, the amount of the aqueous phase 75 to be supplied is 0.5 kg/hr to 500 kg/hr. The aqueous phase 75 to be supplied to the third chamber 33 may be heated in advance. In one example, the aqueous phase 75 to be supplied to the third chamber 33 has a temperature of 30°C to 75°C.

Next, in a state in which the aqueous phase 75 is in contact with the one surface of the second separation membrane 31, a space adjacent to another surface of the second separation membrane 31 is decompressed. Specifically, an inside of the fourth chamber 34 is decompressed via the outlet 34a. The inside of the fourth chamber 34 can be decompressed by, for example, the decompression device provided to the second permeated fluid feed passage 55 or the first permeated fluid feed passage 51. A pressure in the fourth chamber 34 is, for example, 50 kPa or less, and may be 20 kPa or less, 10 kPa or less, 5 kPa or less, 3 kPa or less, or even 2 kPa or less.

Decompressing the inside of the fourth chamber 34 makes it possible to obtain, on the side of the other surface of the second separation membrane 31, the second permeated fluid 76 having a high content of the organic compound C. In other words, the second permeated fluid 76 is supplied to the fourth chamber 34. The second permeated fluid 76 may be a gas or a liquid in the fourth chamber 34. The second permeated fluid 76 is discharged outside the second membrane separation device 30 via the outlet 34a. The second permeated fluid 76 that is a gas is cooled by, for example, the heat exchanger provided to the second permeated fluid feed passage 55 or the first permeated fluid feed passage 51, or the like. Thereby, the second permeated fluid 76 is liquefied to obtain the second permeated fluid 76 that is a liquid.

On the other hand, the content of the organic compound C in the aqueous phase 75 gradually decreases from the inlet 33a toward the outlet 33b in the third chamber 33. The aqueous phase 75 (the second non-permeated fluid 77) processed in the third chamber 33 is discharged outside the second membrane separation device 30 via the outlet 33b. The second non-permeated fluid 77 is typically a liquid.

As described above, the second separation membrane 31 of the second membrane separation device 30 allows the organic compound C contained in the aqueous phase 75 to preferentially permeate therethrough. Accordingly, the second permeated fluid 76 obtained by the operation of the second membrane separation device 30 has a content of the organic compound C higher than that in the aqueous phase 75 to be supplied to the second membrane separation device 30. A ratio of the content (wt%) of the organic compound C in the second permeated fluid 76 with respect to the content (wt%) of the organic compound C in the aqueous phase 75 is, for example, but not particularly limited to, 5 to 50.

Note that the content of the organic compound C in the second non-permeated fluid 77 is lower than the content of the organic compound C in the aqueous phase 75 to be supplied to the second membrane separation device 30 and is, for example, 5 wt% or less, and may be 2 wt% or less or even 1 wt% or less. In one example, the second non-permeated fluid 77 in which the content of the organic compound C is 2 wt% or less tends to be able to inhibit the fermentation by a microorganism from stopping in the aqueous solution S1 even when being mixed with the aqueous solution S1 as the fermented liquid in the later-described mixing step. The lower limit of the content of the organic compound C in the second non-permeated fluid 77 is, for example, but not particularly limited to, 0.001 wt%.

### (Preparation step)

A preparation step is performed as follows, for example. First, the second permeated fluid 76 is supplied to the first permeated fluid feed passage 51 or the separation vessel 25 via the second permeated fluid feed passage 55. Thereby, the first permeated fluid 71 is mixed with the second permeated fluid 76 in the first permeated fluid feed passage 51 or the separation vessel 25, and thereby the aqueous solution S2 can be prepared. In the preparation step, the second permeated fluid 76 that is a liquid may be mixed with the first permeated fluid 71 that is a liquid to prepare the aqueous solution S2, or a gas mixture obtained by mixing the second permeated fluid 76 that is a gas with the first permeated fluid 71 that is a gas may be cooled to prepare the aqueous solution S2. Note that the aqueous solution S2 may be composed only of the first permeated fluid 71 and the second permeated fluid 76, or may contain, in addition to these fluids, the distillate obtained in the later-described distillation step.

### (Mixing step)

The operating method of the present embodiment may further include a mixing step of mixing the second non-permeated fluid 77 with the aqueous solution S1. The mixing step can be performed by supplying the second non-permeated fluid 77 to the aqueous solution feed passage 50 via the second non-permeated fluid discharge passage 56. The mixing step makes it possible to recycle the second non-permeated fluid 77.

The second non-permeated fluid 77 preferably has a content of the organic compound C comparable to that in the aqueous solution S1. In one example, a ratio of the content of the organic compound C in the second non-permeated fluid 77 with respect to the content of the organic compound C in the aqueous solution S1 discharged from the tank 20 is 80% to 120%. In this case, it tends to be possible to suppress sufficiently the energy required to operate the membrane separation system 100.

### (Distillation step)

The operating method of the present embodiment may further include a distillation step of distilling the organic phase obtained in the second separation step. The distillation step is performed as follows, for example. First, the organic phase is supplied to the distillation device 40 via the organic phase feed passage 54. The organic phase can be distilled in the distillation device 40.

In the distillation step, it is possible to further increase the content of the organic compound C in the organic phase by heating the water together with the organic compound C. The distillation step makes it possible to increase the content (purity) of the organic compound C in the organic phase to, for example, 80 wt% or more, 90 wt% or more, 95 wt% or more, or even 99 wt% or more.

The residue obtained by the distillation step (the organic phase after being distilled) is discharged outside the distillation device 40 via the outlet 42. The operating method of the present embodiment makes it possible to obtain the organic compound C at a high purity by recovering this residue.

The distillate obtained by distilling the organic phase has a content of the organic compound C lower than that in the organic phase supplied to the distillation device 40, and a content of water higher than that in the organic phase. The content of the organic compound C in the distillate is, for example, but not particularly limited to, 30 wt% to 70wt%. The distillate obtained by the distillation step may be delivered to the first permeated fluid feed passage 51 via the distillate discharge passage 58 to be mixed with the first permeated fluid 71 and the second permeated fluid 76.

As described above, the membrane separation system 100 of the present embodiment includes the first membrane separation device 10, the separation vessel 25, the second membrane separation device 30, the first permeated fluid feed passage 51, and the second permeated fluid feed passage 55, and is configured in such a manner that the second permeated fluid 76 is supplied to the first permeated fluid feed passage 51 or the separation vessel 25 via the second permeated fluid feed passage 55. The membrane separation system 100 as just mentioned is suitable for efficiently separating the organic compound C from the aqueous solution S1.

For example, the energy required for operation tends to be more suppressed in the membrane separation system 100 of the present embodiment than in the case where the aqueous phase obtained in the separation vessel 25 is distilled by using an additional distillation device instead of the second membrane separation device 30. In the membrane separation system 100 of the present embodiment, it tends to be possible to reduce the amount of carbon dioxide to be emitted during operation by not using the additional distillation device.

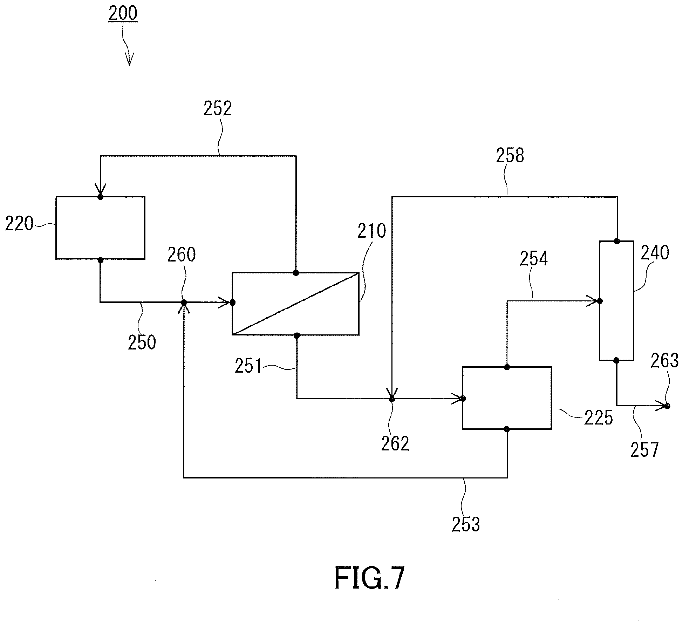

It is possible to recover the organic compound C at a higher recovery rate in the membrane separation system 100 of the present embodiment than in the case where the second membrane separation device 30 is not used and the aqueous phase obtained in the separation vessel 25 is discarded. Moreover, the membrane separation system 100 of the present embodiment is more suitable for suppressing the energy required for operation while reducing the membrane areas of the separation membranes required to separate the aqueous solution S1, etc. than a membrane separation system (such as the later-described membrane separation system 200 of Calculation Example 1 (FIG. 7)) in which the aqueous phase obtained in the separation vessel 25 is mixed with the aqueous solution S1 without using the second membrane separation device 30.

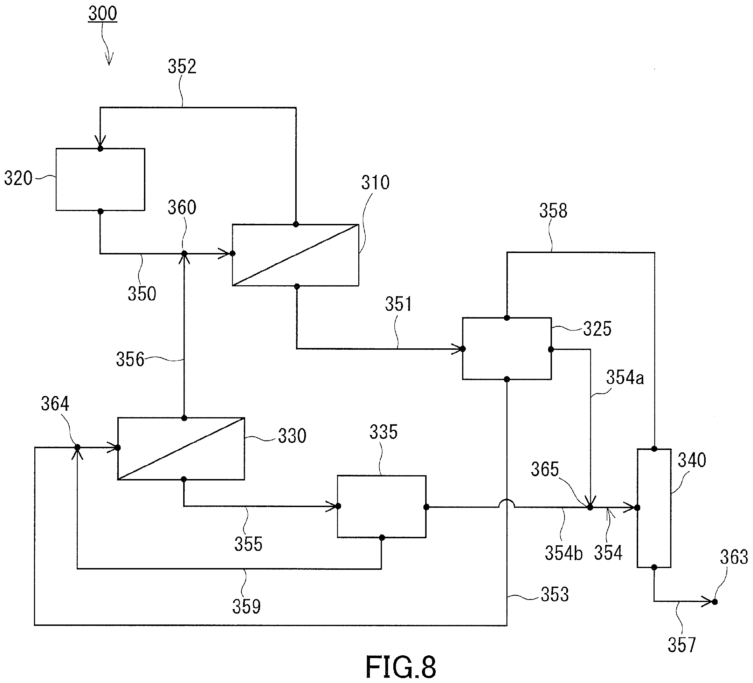

Note that in a membrane separation system (such as the later-described membrane separation system 300 of Calculation Examples 13 and 14 (FIG. 8)) in which the second permeated fluid 76 is not supplied to the first permeated fluid feed passage 51 or the separation vessel 25, it tends to be difficult to adjust the content of the organic compound C in the first permeated fluid to a sufficiently high value. When the content of the organic compound C in the first permeated fluid is low, the first permeated fluid fails to be phase-separated in the separation vessel and thus the organic compound C cannot be efficiently separated from the aqueous solution S1 in some cases.

### [Modification of first membrane separation device]

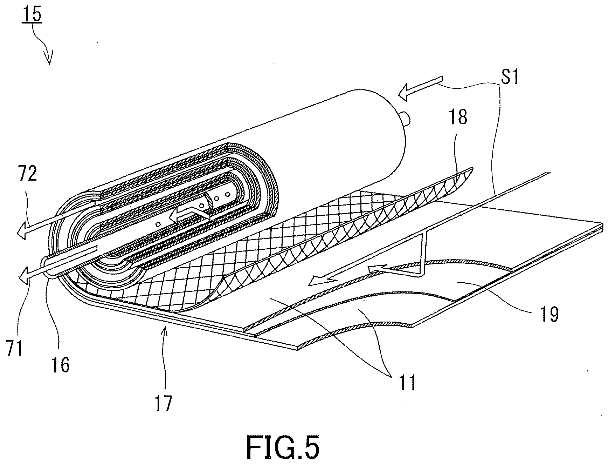

In the membrane separation system 100, the first membrane separation device 10 may be a spiral membrane element, a hollow fiber membrane element, a disk tube membrane element in which a plurality of pervaporation membranes are laminated, a plate-and-flame membrane element, or the like. FIG. 5 shows a spiral membrane element. A first membrane separation device 15 of FIG. 5 includes a central tube 16 and a laminate 17. The laminate 17 includes the first separation membrane 11.

The central tube 16 has a cylindrical shape. The central tube 16 has a surface with a plurality of holes or slits to allow the first permeated fluid 71 to flow into the central tube 16. Examples of a material of the central tube 16 include: a resin such as an acrylonitrile-butadiene-styrene copolymer resin (an ABS resin), a polyphenylene ether resin (a PPE resin), or a polysulfone resin (a PSF resin); and a metal such as stainless steel or titanium. The central tube 16 has an inner diameter in a range of, for example, 20 to 100 mm.

The laminate 17 further includes a feed-side flow passage material 18 and a permeation-side flow passage material 19 in addition to the first separation membrane 11. The laminate 17 is wound around the central tube 16. The first membrane separation device 15 may further include an exterior material (not shown).

As the feed-side flow passage material 18 and the permeation-side flow passage material 19, a resin net, woven fabric, or knitted fabric composed of polyethylene, polypropylene, polyethylene terephthalate (PET), polyphenylene sulfide (PPS), or an ethylene-chlorotrifluoroethylene copolymer (ECTFE) can be used, for example.

A method for operating the first membrane separation device 15 is carried out as follows, for example. First, the aqueous solution S1 is supplied into an end of the wound laminate 17. A space inside the central tube 16 is decompressed. The first permeated fluid 71 having permeated through the first separation membrane 11 of the laminate 17 thereby moves into the central tube 16. The first permeated fluid 71 is discharged outside via the center tube 16. The aqueous solution S1 processed by the first membrane separation device 15 (the first non-permeated fluid 72) is discharged outside from another end of the wound laminate 17.

Note that in the membrane separation system 100, the second membrane separation device 30 may also be a spiral membrane element, a hollow fiber membrane element, a disk tube membrane element in which a plurality of pervaporation membranes are laminated, a plate-and-flame membrane element, or the like. The second membrane separation device 30 may be a spiral membrane element having the same configuration as that of the first membrane separation device 15.

### <Embodiment 2>

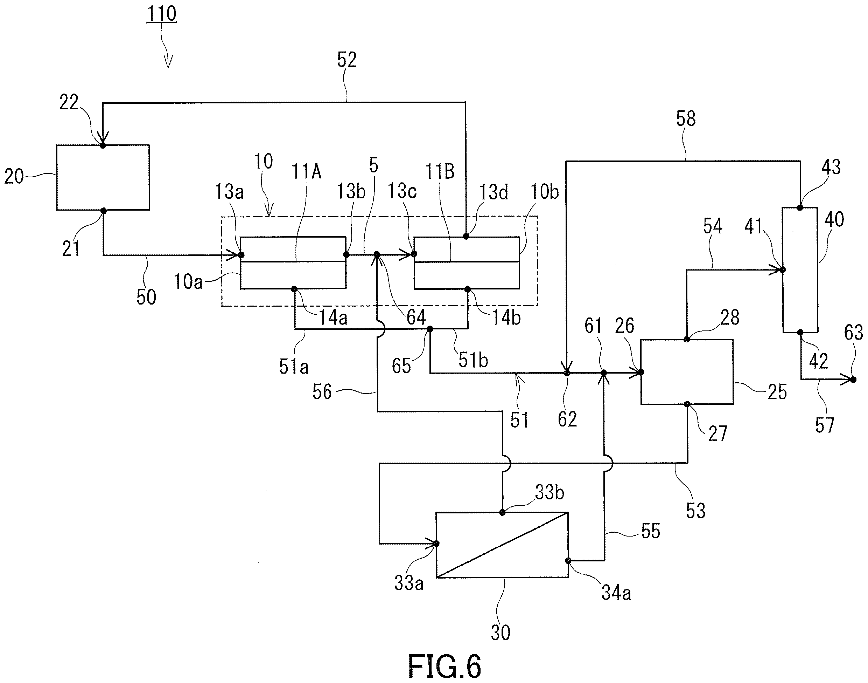

As shown in FIG. 6, in a membrane separation system 110 of Embodiment 2, the first membrane separation device 10 has two membrane separation units 10a and 10b, and a connection passage 5 connecting the two membrane separation units 10a and 10b to each other. The second non-permeated fluid discharge passage 56 is connected to the connection passage 5 at a connecting point 64. The first permeated fluid feed passage 51 has a first portion 51a and a second portion 51b. Except for the above, the membrane separation system 110 has the same configuration as that of the membrane separation system 100 of Embodiment 1. Therefore, the elements common between the membrane separation system 100 of Embodiment 1 and the membrane separation system 110 of the present embodiment are denoted by the same reference numerals, and the description of such elements may be omitted. That is, the description of each of the following embodiments is applicable to the other embodiment unless technical inconsistency occurs. Furthermore, the features of the embodiments may be combined with each other unless technical inconsistency occurs.

Each of the two membrane separation units 10a and 10b has, for example, the same configuration as that of the first membrane separation device 10 of the membrane separation system 100 of Embodiment 1. Specifically, the membrane separation unit 10a has a first separation membrane 11A and further has, for example, a feed space (a first chamber) and a permeation space (a second chamber) separated from each other by the first separation membrane 11A. The aqueous solution feed passage 50 is connected to an aqueous solution inlet (the inlet 13a) of the membrane separation unit 10a. The aqueous solution S1 can be supplied to the feed space of the membrane separation unit 10a via the inlet 13a. Furthermore, the first portion 51a of the first permeated fluid feed passage 51 is connected to a permeated fluid outlet (the outlet 14a) of the membrane separation unit 10a. The first permeated fluid 71 can be discharged outside from the permeation space of the membrane separation unit 10a via the outlet 14a. The connection passage 5 is connected to an aqueous solution outlet (the outlet 13b) of the membrane separation unit 10a. The aqueous solution S1 processed in the membrane separation unit 10a can be discharged outside from the feed space of the membrane separation unit 10a via the outlet 13b.

The membrane separation unit 10b has a first separation membrane 11B and further has, for example, a feed space (a first chamber) and a permeation space (a second chamber) separated from each other by the first separation membrane 11B. The connection passage 5 is connected to an aqueous solution inlet (an inlet 13c) of the membrane separation unit 10b. The aqueous solution S1 can be supplied to the feed space of the membrane separation unit 10b via the inlet 13c. In such a manner, the connection passage 5 connects the feed space of the membrane separation unit 10a to the feed space of the membrane separation unit 10b.

Furthermore, the second portion 51b of the first permeated fluid feed passage 51 is connected to a permeated fluid outlet (an outlet 14b) of the membrane separation unit 10b. The first permeated fluid 71 can be discharged outside from the permeation space of the membrane separation unit 10b via the outlet 14b. The first non-permeated fluid discharge passage 52 is connected to an aqueous solution outlet (an outlet 13d) of the membrane separation unit 10b. The aqueous solution S1 (the first non-permeated fluid 72) processed in the membrane separation unit 10b can be discharged outside from the feed space of the membrane separation unit 10b via the outlet 13d.

The first separation membrane 11A of the membrane separation unit 10a may or may not be the same as the first separation membrane 11B of the membrane separation unit 10b except for its membrane area. A ratio of the membrane area (m²) of the first separation membrane 11A with respect to a total value (m²) of the membrane area of the first separation membrane 11A and a membrane area of the first separation membrane 11B is, for example, but not particularly limited to, 20% to 80%.

Note that the first membrane separation device 10 may further have an additional membrane separation unit other than the membrane separation units 10a and 10b. In the first membrane separation device 10, the number of the membrane separation units is, for example, but not particularly limited to, 2 to 5.

On the first permeated fluid feed passage 51, the second portion 51b joins the first portion 51a at a joining point 65. At the joining point 65, the first permeated fluid 71 discharged from the membrane separation unit 10a can be mixed with the first permeated fluid 71 discharged from the membrane separation unit 10b.

As described above, the second non-permeated fluid discharge passage 56 is connected to the connection passage 5 at the connecting point 64. At the connecting point 64, the second non-permeated fluid 77 can be mixed with the aqueous solution S1 discharged from the membrane separation unit 10a. In the present embodiment, the content of the organic compound C in the second non-permeated fluid 77 is preferably comparable to the content of the organic compound C in the aqueous solution S1 discharged from the membrane separation unit 10a. In one example, a ratio of the content of the organic compound C in the second non-permeated fluid 77 with respect to the content of the organic compound C in the aqueous solution S1 discharged from the membrane separation unit 10a is 80% to 120%. In this case, it tends to be possible to suppress sufficiently the energy required to operate the membrane separation system 110.

### EXAMPLES

Hereinafter, the present invention will be described in more detail by way of measurement examples, but the present invention is not limited to these examples.

### [Production of separation membrane]

First, a separation membrane (a pervaporation membrane) was produced by the following method. A coating liquid was prepared by mixing 1.650 kg (solids concentration: 30 wt%) of a silicone resin (YSR3022 available from Momentive Performance Materials Japan LLC.), 2.805 kg of toluene, 0.495 kg of a high-silica zeolite (HiSiv 3000 available from UNION SHOWA K.K.), 0.0495 kg of a silicone curing catalyst (YC6831 available from Momentive Performance Materials Japan LLC.), and 0.0495 kg of acetylacetone as a curing retardant. Next, the coating liquid was applied onto a porous support (RS-50 available from Nitto Denko Corporation) having a thickness of 150 µm to obtain a coating (thickness: 500 µm). The coating was heated at 90°C for 4 minutes and then dried to produce a separation functional layer having a thickness of 50 µm. The weight ratio between the silicone resin and the high-silica zeolite was 50:50 in the separation functional layer. Thus, a separation membrane was obtained.

### (Calculation Example 1)

The operation of a membrane separation system 200 shown in FIG. 7 was simulated using the produced separation membrane. The membrane separation system 200 included a first membrane separation device 210, a tank 220, a separation vessel 225, a distillation device 240, an aqueous solution feed passage 250, a first permeated fluid feed passage 251, an aqueous phase feed passage 253, etc. The membrane separation system 200 had the same configuration as that of the membrane separation system 100 shown in FIG. 1, except that the second membrane separation device was omitted and the aqueous phase feed passage 253 was connected to the aqueous solution feed passage 250. In the membrane separation system 200, it was assumed to use the above-mentioned separation membrane as the first separation membrane included in the first membrane separation device 210.

In Calculation Example 1, the energy required to operate the membrane separation system 200 and the membrane area of the first separation membrane were calculated when the following assumptions were satisfied. Symmetry, a process modeling software available from Schlumberger Ltd., was used for the calculations.

### • Assumptions

The aqueous solution S1 to be delivered from the tank 220 is an aqueous solution containing n-butanol at a content of 0.8 wt%.

The content of the n-butanol in the non-permeated fluid discharged from the first membrane separation device 210 is 0.08 wt%.

The content of the n-butanol in the aqueous phase generated in the separation vessel 225 is 7 wt%.

The content of the n-butanol in the organic phase generated in the separation vessel 225 is 80 wt%.

As for the residue obtained in the distillation device 240, the recovery amount of the n-butanol is 250 kg/h, the content (purity) of the n-butanol is 99.5 wt%, and the recovery rate of the n-butanol is 90%.

In the operation of the first membrane separation device 210, it was assumed to obtain the permeated fluid that was a gas by decompressing the inside of the permeation space while heating the aqueous solution S1, and to cool the obtained permeated fluid in the chiller to liquefy it.

### (Calculation Examples 2 to 6)

The operation of the membrane separation system 100 shown in FIG. 1 was simulated using the produced separation membrane. Specifically, it was assumed to use the produced separation membrane for each of the first separation membrane 11 included in the first membrane separation device 10 and the second separation membrane 31 included in the second membrane separation device 30 in the membrane separation system 100.

In Calculation Examples 2 to 6, the energy required to operate the membrane separation system 100 and the membrane area of the first separation membrane 11 were calculated when the following assumptions were satisfied. Symmetry, a process modeling software available from Schlumberger Ltd., was used for the calculations.

### • Assumptions

The aqueous solution S1 to be delivered from the tank 20 is an aqueous solution containing n-butanol at a content of 0.8 wt%.

The content of the n-butanol in the first non-permeated fluid 72 discharged from the first membrane separation device 10 is 0.08 wt%.

The content of the n-butanol in the aqueous phase generated in the separation vessel 25 is 7 wt%.

The content of the n-butanol in the organic phase generated in the separation vessel 25 is 80 wt%.

As for the residue obtained in the distillation device 40, the recovery amount of the n-butanol is 250 kg/h, the content (purity) of the n-butanol is 99.5 wt%, and the recovery rate of the n-butanol is 90%.

In the operation of the first membrane separation device 10, it was assumed to obtain the first permeated fluid 71 that was a gas by decompressing the inside of the permeation space while heating the aqueous solution S1, and to cool the obtained first permeated fluid 71 in the chiller to liquefy it. Similarly, in the operation of the second membrane separation device 30, it was assumed to obtain the second permeated fluid 76 that was a gas by decompressing the inside of the permeation space while heating the aqueous phase, and to cool the obtained second permeated fluid 76 in the chiller to liquefy it. Among Calculation Examples 2 to 6, the membrane area of the second separation membrane 31 was varied and the impact thereof was observed.

### (Calculation Examples 7 to 12)

The operation of the membrane separation system 110 shown in FIG. 6 was simulated using the produced separation membrane. Specifically, it was assumed to use the produced separation membrane for each of the first separation membrane 11A and the first separation membrane 11B included in the first membrane separation device 10 and the second separation membrane 31 included in the second membrane separation device 30 in the membrane separation system 110.

In Calculation Examples 7 to 12, the energy required to operate the membrane separation system 110 and the membrane areas of the first separation membranes 11A and 11B were calculated when the following assumptions were satisfied. Symmetry, a process modeling software available from Schlumberger Ltd., was used for the calculations.

### • Assumptions

The aqueous solution S1 to be delivered from the tank 20 is an aqueous solution containing n-butanol at a content of 0.8 wt%.

The content of the n-butanol in the aqueous solution S1 discharged from the membrane separation unit 10a is 0.2 wt%.

The content of the n-butanol in the first non-permeated fluid 72 discharged from the membrane separation unit 10b is 0.08 wt%.

The content of the n-butanol in the aqueous phase generated in the separation vessel 25 is 7 wt%.

The content of the n-butanol in the organic phase generated in the separation vessel 25 is 80 wt%.

As for the residue obtained in the distillation device 40, the recovery amount of the n-butanol is 250 kg/h, the content (purity) of the n-butanol is 99.5 wt%, and the recovery rate of the n-butanol is 90%.

In the operation of the first membrane separation device 10, it was assumed to obtain the first permeated fluid 71 that was a gas by decompressing the inside of the permeation space while heating the aqueous solution S1, and to cool the obtained first permeated fluid 71 that was a gas in the chiller to liquefy it. Similarly, in the operation of the second membrane separation device 30, it was assumed to obtain the second permeated fluid 76 that was a gas by decompressing the inside of the permeation space while heating the aqueous phase, and to cool the obtained second permeated fluid 76 in the chiller to liquefy it. Among Calculation Examples 7 to 12, the membrane area of the second separation membrane 31 was varied and the impact thereof was observed.

**[Table 1]**

| | | Calculation Example 1 | Calculation Example 2 | Calculation Example 3 | Calculation Example 4 | Calculation Example 5 | Calculation Example 6 |
|---|---|---|---|---|---|---|---|
| Second membrane separation device | Present/Absent | Absent | Present | Present | Present | Present | Present |
| | Membrane area of second separation membrane (m²) | - | 3172 | 6223 | 9439 | 11109 | 13232 |
| | Energy consumed (kW) | - | 66 | 121 | 169 | 192 | 220 |
| | Concentration of BuOH in second non-permeated fluid (wt%) | - | 4.6 | 2.6 | 1.2 | 0.74 | 0.46 |
| Energy consumed by first membrane separation device (kW) | | 3514 | 3214 | 2891 | 2652 | 2549 | 2536 |
| Energy consumed by chiller(s) (kW) | | 3631 | 3390 | 3113 | 2916 | 2832 | 2849 |
| Total value of membrane areas (m²) (*1) | | 168000 | 154315 | 140711 | 130191 | 125417 | 124939 |
| Energy required for operating the system (kW) | | 7161 | 6806 | 6261 | 5874 | 5708 | 5741 |

| | | | | | | | |
|---|---|---|---|---|---|---|---|
| (*1) The total value of the membrane area of the first separation membrane and that of the second separation membrane. | | | | | | | |

**[Table 2]**

| | | Calculation Example 7 | Calculation Example 8 | Calculation Example 9 | Calculation Example 10 | Calculation Example 11 | Calculation Example 12 |
|---|---|---|---|---|---|---|---|
| Second membrane separation device | Membrane area of second separation membrane (m²) | 10605 | 13532 | 15146 | 15930 | 17383 | 28386 |
| | Energy consumed (kW) | 190 | 225 | 244 | 252 | 272 | 405 |
| | Concentration of BuOH in second non-permeated fluid (wt%) | 1.2 | 0.44 | 0.25 | 0.19 | 0.14 | 0.006 |
| Energy consumed by first membrane separation device (kW) | | 2973 | 2593 | 2474 | 2418 | 2460 | 2321 |
| Energy consumed by chiller(s) (kW) | | 3271 | 2914 | 2810 | 2761 | 2825 | 2819 |
| Total value of membrane areas (m²) (*1) | | 157442 | 141169 | 136877 | 134880 | 138315 | 142504 |
| Energy required for operating the system (kW) | | 6572 | 5869 | 5666 | 5569 | 5695 | 5682 |

| | | | | | | | |
|---|---|---|---|---|---|---|---|
| (*1) The total value of the membrane area of the first separation membrane 11A, that of the first separation membrane 11B, and that of the second separation membrane 31. | | | | | | | |

In Tables 1 and 2, the energy consumed by the membrane separation device reflects the energy required to heat the aqueous solution S1 or the aqueous phase to be supplied to the membrane separation device, and the like. This energy is determined in accordance with the membrane area of the separation membrane, the energy required to evaporate the water and n-butanol, and the like. The energy consumed by the membrane separation device tends to increase as the amount of water contained in the permeated fluid increases. The energy consumed by the chiller(s) reflects the energy required to cool and liquefy the permeated fluid that is a gas and that is obtained in each membrane separation device, and the like. This energy is determined in accordance with the flow rate of the permeated fluid, the energy required to liquefy the water and n-butanol, and the like. The energy consumed by the chiller(s) tends to increase as the amount of the water contained in the permeated fluid increases. The energy required to operate the system reflects not only the energies consumed by each membrane separation device and chiller but also the energy consumed by the distillation device, and the like.

As can be seen from Tables 1 and 2, the membrane separation system 100 shown in FIG. 1 and the membrane separation system 110 shown in FIG. 6 are more suitable for suppressing the energy required for operation while reducing the membrane areas of the separation membranes required to separate the aqueous solution S1, etc. than the membrane separation system 200 shown in FIG. 7. This result reveals that the membrane separation system of the present embodiment is suitable for efficiently separating a volatile organic compound from an aqueous solution containing the organic compound.

Furthermore, in Calculation Example 5, in which the content of the n-butanol in the second non-permeated fluid 77 was comparable to the content (0.8 wt%) of the n-butanol in the aqueous solution S1 to be delivered from the tank 20, the energy required to operate the membrane separation system 100 decreased most among Calculation Examples 2 to 6 using the membrane separation system 100 shown in FIG. 1. In Calculation Example 5, the membrane areas of the separation membranes (the total value of the membrane areas) required to separate the aqueous solution S1, etc. decreased sufficiently as well.

Similarly, in Calculation Example 10, in which the content of the n-butanol in the second non-permeated fluid 77 was comparable to the content (0.2 wt%) of the n-butanol in the aqueous solution S1 discharged from the membrane separation unit 10a, the energy required to operate the membrane separation system 110 decreased most among Calculation Examples 7 to 12 using the membrane separation system 110 shown in FIG. 6. In Calculation Example 10, the membrane areas of the separation membranes (the total value of the membrane areas) required to separate the aqueous solution S1, etc. decreased sufficiently as well.

### (Calculation Examples 13 and 14)

Next, the operation of a membrane separation system 300 shown in FIG. 8 was simulated using the produced separation membrane. The membrane separation system 300 included a first membrane separation device 310, a tank 320, a separation vessel 325, a second membrane separation device 330, an additional separation vessel 335, a distillation device 340, an aqueous solution feed passage 350, a first permeated fluid feed passage 351, an aqueous phase feed passage 353, an organic phase feed passage 354, a second permeated fluid feed passage 355, a distillate discharge passage 358, etc. The membrane separation system 300 had the same configuration as that of the membrane separation system 100 shown in FIG. 1, except that the membrane separation system 300 included the additional separation vessel 335, the second permeated fluid feed passage 355 was connected to the additional separation vessel 335, and so on. It was assumed to use the produced separation membrane for each of the first separation membrane included in the first membrane separation device 310 and the second separation membrane included in the second membrane separation device 330 in the membrane separation system 300.

The separation vessel 335 is a vessel for phase-separating the second permeated fluid discharged from the second membrane separation device 330. When the second permeated fluid that is a liquid is left at rest in the additional separation vessel 335, the second permeated fluid is phase-separated into an aqueous phase and an organic phase. The membrane separation system 300 is connected to the additional separation vessel 335 and the aqueous phase feed passage 353, and further includes an additional aqueous phase feed passage 359 for supplying the aqueous phase from the additional separation vessel 335 to the aqueous phase feed passage 353. The additional aqueous phase feed passage 359 is connected to the aqueous phase feed passage 353 at a connecting point 364.

In the membrane separation system 300, the organic phase feed passage 354 has a first portion 354a and a second portion 354b. The second portion 354b is a passage connected to the additional separation vessel 335 and the distillation device 340 and configured to supply the organic phase from the additional separation vessel 335 to the distillation device 340. The first portion 354a is a passage connected to the separation vessel 325 and the second portion 354b and configured to supply the organic phase from the separation vessel 325 to the second portion 354b. The first portion 354a is connected to the second portion 354b at a connecting point 365.

In the membrane separation system 300, the distillate discharge passage 358 is connected not to the first permeated fluid feed passage 351 but to the separation vessel 325.

In Calculation Examples 13 and 14, it was tried to calculate the energy required to operate the membrane separation system 300 and the membrane area of the first separation membrane 11 when the following assumptions held. However, in Calculation Examples 13 and 14, the content of the n-butanol in the first permeated fluid discharged from the first membrane separation device 310 was less than 7 wt%. In this case, the first permeated fluid failed to be phase-separated in the separation vessel 325, and thus it was impossible to simulate properly the operation of the membrane separation system 300.

### • Assumptions

The aqueous solution S1 to be delivered from the tank 320 is an aqueous solution containing n-butanol at a content of 0.8 wt%.

The content of the n-butanol in the first non-permeated fluid discharged from the first membrane separation device 310 is 0.08 wt%.

The content of the n-butanol in the aqueous phase generated in each of the separation vessel 325 and the additional separation vessel 335 is 7 wt%.

The content of the n-butanol in the organic phase generated in each of the separation vessel 325 and the additional separation vessel 335 is 80 wt%.

As for the residue obtained in the distillation device 40, the recovery amount of the n-butanol is 250 kg/h, the content (purity) of the n-butanol is 99.5 wt%, and the recovery rate of the n-butanol is 90%.

**[Table 3]**

| | | Calculation Example 13 | Calculation Example 14 |
|---|---|---|---|
| Second membrane separation device | Membrane area of second separation membrane (m²) | 13399 | 11351 |
| | Concentration of BuOH in second non-permeated fluid (wt%) | 0.3 | 0.7 |
| Concentration of BuOH in first permeated fluid | | 6.6 | 6.8 |
| Phase separation in separation vessel | | Impossible | Impossible |

As can be seen from Table 3, in the case where the second permeated fluid discharged from the second membrane separation device 330 is not supplied to the first permeated fluid feed passage 351 or the separation vessel 325, it tends to be difficult to adjust the content of the organic compound C in the first permeated fluid to a sufficiently high value. When the content of the organic compound C in the first permeated fluid is low, the first permeated fluid fails to be phase-separated in the separation vessel 325 and thus the organic compound C cannot be efficiently separated from the aqueous solution S1 in some cases.

In the membrane separation system 300, there is a possibility that the content of the organic compound C in the first permeated fluid can be increased by decreasing the membrane area of the second separation membrane to be less than that in Calculation Example 14. In this case, however, the energy required to operate the membrane separation system 300 and the membrane area of the first separation membrane tend to increase significantly.

### (Calculation Example 15)

The operation of the membrane separation system 200 shown in FIG. 7 was simulated under operating conditions different from those in Calculation Example 1, and the energy required to operate the membrane separation system 200 and the membrane area of the first separation membrane were calculated. Symmetry, a process modeling software available from Schlumberger Ltd., was used for the calculations. UNIFAC was used as the thermodynamic model. The conditions for supplying the aqueous solution S1, and the organic phase and the aqueous phase in the separation vessel 225 were set as follows. Note that in Calculation Example 15, it was assumed to discharge the non-permeated fluid outside the membrane separation system 200 without connecting the first non-permeated fluid discharge passage 252 to the tank 220. Furthermore, in Calculation Example 15, it was assumed to recover the organic phase generated in the separation vessel 225 without operating the distillation device 240.

### [Conditions for supplying aqueous solution S1]

Flow rate: 31250 kg/h
Temperature: 20°C
Pressure: 1 atm
Composition: An aqueous solution containing methyl ethyl ketone (MEK) at a content of 1 wt%.

### [Organic phase in separation vessel]

Flow rate: 336 kg/h
Temperature: 40°C
Composition: The content of the MEK was 88.2 wt%.
Recovery rate of MEK: 95%

### [Aqueous phase in separation vessel]

Flow rate: 1051 kg/h
Temperature: 40°C
Composition: The content of the MEK was 21.5 wt%.

In the operation of the first membrane separation device 210, it was assumed to obtain the permeated fluid that was a gas by decompressing the inside of the permeation space to 1.5 kPa while heating the aqueous solution S1 to 40°C. It was assumed that the permeated fluid that was a gas had a flow rate of 1387 kg/h and the content of the MEK therein was 37.7 wt%. It was assumed that the non-permeated fluid had a flow rate of 30914 kg/h and the content of the MEK therein was 0.05 wt%. It was assumed to cool the obtained permeated fluid to -13°C in the heat exchanger (the chiller) to liquefy it, and furthermore to heat the permeated fluid that was a liquid to 40°C using another heat exchanger, and then supply the permeated fluid that was a liquid to the separation vessel 225.

### (Calculation Example 16)

The operation of the membrane separation system 100 shown in FIG. 1 was simulated under operating conditions different from those in Calculation Example 2, and the energy required to operate the membrane separation system 100 and the membrane area of the first separation membrane were calculated. Symmetry, a process modeling software available from Schlumberger Ltd., was used for the calculations. UNIFAC was used as the thermodynamic model. The conditions for supplying the aqueous solution S1, and the organic phase and the aqueous phase in the separation vessel 25 were set as follows. Note that in Calculation Example 16, it was assumed to discharge the first non-permeated fluid outside the membrane separation system 100 without connecting the first non-permeated fluid discharge passage 52 to the tank 20. Furthermore, in Calculation Example 16, it was assumed to recover the organic phase generated in the separation vessel 25 without operating the distillation device 40.

### [Conditions for supplying aqueous solution S1]

Flow rate: 31250 kg/h
Temperature: 20°C
Pressure: 1 atm
Composition: An aqueous solution containing methyl ethyl ketone (MEK) at a content of 1 wt%.

### [Organic phase in separation vessel]

Flow rate: 336 kg/h
Temperature: 40°C
Composition: The content of the MEK was 88.2 wt%.
Recovery rate of MEK: 95%

### [Aqueous phase in separation vessel]

Flow rate: 906 kg/h
Temperature: 40°C
Composition: The content of the MEK was 21.5 wt%.

In the operation of the first membrane separation device 10, it was assumed to obtain the first permeated fluid that was a gas by decompressing the inside of the permeation space to 1.5 kPa while heating the aqueous solution S1 to 40°C. It was assumed that the first permeated fluid that was a gas had a flow rate of 1037 kg/h and the content of the MEK therein was 29.3 wt%. It was assumed that the first non-permeated fluid had a flow rate of 30914 kg/h and the content of the MEK therein was 0.05 wt%. It was assumed to mix the obtained first permeated fluid with the later-described second permeated fluid, cool the mixture to -13°C in the heat exchanger (the chiller) to liquefy it, and furthermore to heat the obtained aqueous solution S2 to 40°C using another heat exchanger, and then supply the aqueous solution S2 to the separation vessel 25. Furthermore, in the operation of the second membrane separation device 30, it was assumed to obtain the second permeated fluid that was a gas by decompressing the inside of the permeation space to 1.5 kPa while heating the aqueous phase to 40°C. It was assumed that the second permeated fluid that was a gas had a flow rate of 205 kg/h and the content of the MEK therein was 91.7 wt%. It was assumed that the second non-permeated fluid had a flow rate of 701 kg/h and the content of the MEK therein was 1 wt%. The aqueous solution S2 obtained by mixing the first permeated fluid with the second permeated fluid had a flow rate of 1242 kg/h and the content of the MEK therein was 39.6 wt%.

### (Calculation Example 17)

The energy required was calculated in the case where the aqueous solution S1 was supplied to a distillation column under the conditions for supplying the aqueous solution S1 in Calculation Examples 15 and 16 and the distillation was performed in the distillation column. Symmetry, a process modeling software available from Schlumberger Ltd., was used for the calculations. UNIFAC was used as the thermodynamic model. The conditions for the distillation, and the distillate and the residue (the aqueous solution S1 after being distilled) to be obtained by the distillation were set as follows. Note that it was assumed to recover the distillate in Calculation Example 17.

### [Conditions for distillation]

Number of stages: 8
Charging port for aqueous solution S1: Sixth stage from the top
Reflux ratio: 1.56

### [Distillate]

Flow rate: 336 kg/h
Temperature: 73.5°C
Composition: The content of the MEK was 88.2 wt%.
Recovery rate of MEK: 95%

### [Residue (aqueous solution S after being distilled)]

Flow rate: 30914 kg/h
Temperature: 99.5°C
Composition: The content of the MEK was 0.05 wt%.

In Calculation Example 17, it was assumed to heat the aqueous solution S1 to 40°C before supplying it to the distillation column. It was assumed to raise the temperature of the aqueous solution S1 in the distillation column by a heat source, such as steam. It was assumed to cool the distillate using the water in a cooling tower.

**[Table 4]**

| | | Calculation Example 15 | Calculation Example 16 | Calculation Example 17 |
|---|---|---|---|---|
| Second membrane separation device | Present/Absent | Absent | Present | Absent |
| | Membrane area of second separation membrane (m²) | - | 158 | - |
| | Energy consumed (kW) | - | 36 | - |
| | Concentration of MEK in second non-permeated fluid (wt%) | - | 1 | - |
| Energy consumed by first membrane separation device (kW) | | 645 | 529 | - |
| Energy consumed by chiller(s) (kW) | | 710 | 623 | - |
| Total value of membrane areas (m²) (*1) | | 8791 | 7664 | - |
| Energy required for operating the system (kW) | | 2094 | 1918 | 3161 |

| | | | | |
|---|---|---|---|---|
| (*1) The total value of the membrane area of the first separation membrane and that of the second separation membrane. | | | | |

As can be seen from Table 4, the energy required for operation was a smaller value in Calculation Example 16 using the membrane separation system 100 shown in FIG. 1 than in Calculation Examples 15 and 17. Also, comparison between Calculation Examples 15 and 16 reveals that the membrane separation system 100 makes it possible to reduce the membrane areas of the separation membranes required for separation of the aqueous solution S1, etc. These results reveal that the membrane separation system of the present embodiment is suitable for efficiently separating a volatile organic compound from an aqueous solution containing the organic compound.

### INDUSTRIAL APPLICABILITY

The membrane separation system of the present embodiment is suitable for efficiently separating a volatile organic compound from an aqueous solution containing the organic compound.

## Claims

1. A membrane separation system comprising:
a first membrane separation device having a first separation membrane that allows a first permeated fluid to be generated from an aqueous solution S1 containing a volatile organic compound, the first permeated fluid having a content of the organic compound higher than that in the aqueous solution S1;
a separation vessel for phase-separating an aqueous solution S2 containing the first permeated fluid into an aqueous phase having a content of water higher than that in the aqueous solution S2 and containing the organic compound and an organic phase having a content of the organic compound higher than that in the aqueous solution S2;
a second membrane separation device having a second separation membrane that allows a second permeated fluid to be generated from the aqueous phase, the second permeated fluid having a content of the organic compound higher than that in the aqueous phase;
a first permeated fluid feed passage connected to the first membrane separation device and the separation vessel and configured to supply the first permeated fluid from the first membrane separation device to the separation vessel; and
a second permeated fluid feed passage connected to the second membrane separation device and at least one selected from the group consisting of the first permeated fluid feed passage and the separation vessel and configured to supply the second permeated fluid from the second membrane separation device to the first permeated fluid feed passage and/or the separation vessel.

2. The membrane separation system according to claim 1, wherein the aqueous solution S2 is prepared by mixing the first permeated fluid with the second permeated fluid.

3. The membrane separation system according to claim 1 or 2, further comprising a tank for storing the aqueous solution S1 to be supplied to the first membrane separation device.

4. The membrane separation system according to claim 3, wherein
the organic compound is a fermented product generated by a microorganism, and
the tank is a fermenter for generating the organic compound.

5. The membrane separation system according to claim 3, wherein
the first separation membrane allows a first non-permeated fluid to be generated from the aqueous solution S1, the first non-permeated fluid having a content of the organic compound lower than that in the aqueous solution S1,
the membrane separation system further comprises a first non-permeated fluid discharge passage connected to the first membrane separation device and configured to discharge the first non-permeated fluid from the first membrane separation device, and
the first non-permeated fluid discharge passage is connected to the tank.

6. The membrane separation system according to claim 1, wherein
the second separation membrane allows a second non-permeated fluid to be generated from the aqueous phase, the second non-permeated fluid having a content of the organic compound lower than that in the aqueous phase, and
the membrane separation system further comprises a second non-permeated fluid discharge passage connected to the second membrane separation device and configured to discharge the second non-permeated fluid from the second membrane separation device.

7. The membrane separation system according to claim 6, further comprising an aqueous solution feed passage connected to the first membrane separation device and configured to supply the aqueous solution S1 to the first membrane separation device, wherein
the second non-permeated fluid discharge passage is connected to the aqueous solution feed passage.

8. The membrane separation system according to claim 6, wherein
the first membrane separation device has two membrane separation units and a connection passage connecting the two membrane separation units to each other, and
the second non-permeated fluid discharge passage is connected to the connection passage.

9. The membrane separation system according to claim 6, wherein a content of the organic compound in the second non-permeated fluid is 2 wt% or less.

10. The membrane separation system according to claim 1, further comprising a distillation device that distills the organic phase and generates a distillate having a content of the organic compound lower than that in the organic phase.

11. The membrane separation system according to claim 10, further comprising a distillate discharge passage connected to the distillation device and configured to discharge the distillate from the distillation device, wherein
the distillate discharge passage is connected to at least one selected from the group consisting of the first permeated fluid feed passage and the separation vessel.

12. The membrane separation system according to claim 1, wherein at least one selected from the group consisting of the first separation membrane and the second separation membrane is a pervaporation membrane.

13. The membrane separation system according to claim 1, wherein the organic compound is at least one selected from the group consisting of an alcohol, ketone, and ester.

14. A method for operating a membrane separation system including a first membrane separation device having a first separation membrane, a separation vessel, and a second membrane separation device having a second separation membrane, the method comprising:
generating, with the first separation membrane, a first permeated fluid from an aqueous solution S1 containing a volatile organic compound, the first permeated fluid having a content of the organic compound higher than that in the aqueous solution S1;
phase-separating, with the separation vessel, an aqueous solution S2 containing the first permeated fluid into an aqueous phase having a content of water higher than that in the aqueous solution S2 and containing the organic compound and an organic phase having a content of the organic compound higher than that in the aqueous solution S2;
generating, with the second separation membrane, a second permeated fluid from the aqueous phase, the second permeated fluid having a content of the organic compound higher than that in the aqueous phase; and
preparing the aqueous solution S2 by mixing the first permeated fluid with the second permeated fluid.
